# EUROPEAN PATENT APPLICATION

(11) **EP 3 622 945 A1**
(43) Date of publication of application: **18.03.2020**
(21) Application number: 18798924.9
(22) Date of filing: 07.05.2018
(51) Int. Cl.: A61K 8/19, A61K 8/29, A61K 8/06, A61K 8/92, A61K 8/73, A61K 8/67, A61Q 1/02

(54) **COSMETIC BODY COMPOSITION, BODY LOTION, USE AND COSMETIC METHOD FOR EVENING OUT SKIN TONE**

(30) Priority: 12.05.2017 BR 102017010071
(71) Applicant: Natura Cosméticos S.A., 05106-000 São Paulo - SP (BR)
(72) Inventor: DE SOUZA FERREIRA GARCIA, Cinthia, CEP-05106-000 São Paulo - SP (BR); BRITO SILVA, Lais, CEP-05106-000 São Paulo - SP (BR)
(74) Representative: Simmons & Simmons
(86) International application number: PCT/BR2018/050150
(87) International publication number: WO 2018/205001

(57) **Abstract**

The present invention relates to cosmetic body compositions, particularly in the form of a body moisturizer, which comprise particles having encapsulated pigments which are capable of evening the body's skin tone by providing a pantyhose effect, improving its overall appearance, hydration, radiance and strengthening its barrier. It further refers to the cosmetic use and the cosmetic method of evening the skin tone.

## Description

### Field of the Invention

The present invention relates to cosmetic body compositions, particularly in the form of a body moisturizer, which comprise particles having encapsulated pigments which are capable of evening the body's skin tone, improving its overall appearance, providing a pantyhose effect, hydration, radiance and strengthening its barrier. It further refers to the cosmetic use and the cosmetic method of evening the skin tone.

### Background of the invention

The current concept of beauty sought by most people nowadays is that of a young skin without any spots or wrinkles. However, aging, sun exposure, stress and pollution cause the skin to undergo gradual changes, which characterize the signs of skin aging, particularly by reducing the tone evenness, appearance of spots, dryness, and the like.

The daily use of makeup in current days has directly influenced the need for the development of products containing functional active ingredients, providing treatment while masking imperfections of makeup skin.

Makeup and treatment products have been developed for the face, particularly those designated as "Blemish Balm Cream" or "BB cream" or antispot balm. In general, these products adapt to the various types of facial skin, especially the most sensitive ones, having lighter formula and unlike other products, further containing functional active ingredients that provide a more uniformly textured, revitalized skin and even younger skin over time.

However, availability of body products having these features is still limited.

Thus, there remains a need for multifunctional cosmetic compositions for the body skin which can mask imperfections and provide cosmetic treatment.

### Brief description of the figures

Figure 1 shows the skin hydration kinetics by corneometry, showing the percentage of hydration improvement provided by the cosmetic body composition according to the present invention as compared to the baseline skin condition (prior to application of the composition).
Figure 2 shows the mean values of the variation in transepidermal skin water loss (mean ± SD, n = 27), showing the hydration performance of the cosmetic body composition according to the present invention for up to 48 hours.
Figure 3 shows the skin color evenness provided by the use of the cosmetic composition according to the present invention. Skin color evenness was calculated as the percentage reduction in skin color variation after application relative to baseline.
Figure 4 shows the variation in skin color at the baseline and after 15 minutes of the application of the cosmetic body composition according to the present invention.
Figure 5 shows results of total color variation ΔE*94 (calculated relative to the standard sample) of fabric samples after application of the cosmetic body composition according to the present invention and after washing.
Figure 6 shows the percentage of acceptance by volunteers in terms of the attributes assessed after using the cosmetic body composition according to the present invention.

### Description of the invention

The present invention refers to multifunctional cosmetic body compositions that are capable of evening the body skin tone, in addition to improving its overall appearance, promoting hydration, radiance, strengthening its barrier while not staining the clothes.

Thus, in a first aspect, the present invention relates to a cosmetic body composition comprising:
(a) at least one ingredient that provides essential nutrients to the skin; and
(b) at least one encapsulated pigment.

The ingredient that provides essential nutrients to the skin provides softness and vitality to the skin and can be selected from emollients, humectants, conditioners and/or antioxidants, being particularly C₈-C₁₀ triglyceride, C₁₆-C₁₈ triglyceride, tocopheryl acetate, squalene and/or glycerin. Said ingredient is present in an amount of from 0.1 to 20% by weight relative to the total weight of the composition.

Without any limitation, the C₈-C₁₀ triglyceride according to the present invention can be preferably selected from caprylic/capric triglyceride and the C₁₆-C₁₈ triglyceride can be selected from Olus oil (e.g., Cegesoft PS 6) or microalgae. These ingredients contribute to skin radiance.

In a preferred embodiment, the cosmetic body composition according to the present invention comprises a combination of ingredients to provide lasting nutrition, which ingredients consist of C₈-C₁₀ triglyceride, C₁₆-C₁₈ triglyceride, tocopheryl acetate, squalene and glycerin.

The encapsulated pigment according to the present invention, besides providing immediate pigmentation also promotes an even effect. Said ingredient may be selected from one or more of CI 77491, CI 77492, CI 77499 and/or CI 77891. Also, in a particular embodiment, the ingredient may further comprise cellulose acetate, ammonium methacrylate copolymer, magnesium stearate and/or propylene glycol stearate.

The encapsulated pigment is present in an amount of from 0.1 to 3% by weight relative to the total weight of the composition.

In a preferred embodiment, the cosmetic body composition according to the present invention comprises a mixture of three pigments CI 77491, CI 77492 and CI 77499, individually encapsulated with a second pigment CI 77891 and cellulose acetate, ammonium methacrylate copolymer, magnesium stearate and propylene glycol stearate. The mixture of pigment provides a composition that is suitable for skins of different tones.

In a second aspect, the present invention relates to a body moisturizer comprising the cosmetic body composition according to the present invention together with cosmetically acceptable excipients.

Cosmetically acceptable excipients may be selected from those known in the art, for example those cited in the CTFA - International Cosmetic Ingredient Dictionary, 16th edition, 2016.

In another aspect, the present invention further relates to the use of the cosmetic body composition as described herein in the manufacture of a body moisturizer useful to moisturize, strengthen the barrier and/or even the tone and radiance of the skin.

In yet another aspect, the present invention relates to the method of evening the skin tone which consists of applying on the body region in need thereof a cosmetically effective amount of the cosmetic composition according to the present invention until rupture of the pigment particles and absorption of the composition into the skin.

Among the main distinguishing features of the composition according to the present invention is the presence of encapsulated pigments which, upon application to the skin, rupture and transfer thereto a slight tone and easily perceived evenness that can also be considered as a "pantyhose" effect", in addition to moisturizing the skin for up to 48h which, in a particular embodiment, is provided by combining emollients such as squalane, which is also naturally produced by the skin, with a powerful humectant, i.e., glycerin. According to the present invention, "pantyhose effect" means masking of skin imperfections immediately after application of the product, as demonstrated by image analysis, being comparable to the effect of masking leg skin imperfections conferred by semi-transparent socks, also known as pantyhoses.

Emollients present in the cosmetic body composition according to the present invention further stimulate the strengthening of the skin barrier, that is, they reinforce the lipid cement naturally produced by the skin. The consequence of the interaction of said rich formulation with the skin is softness and nutrition, as well as radiance and evenness.

The following examples, without limitation, illustrate the compositions according to the present invention as well as their effects, as described herein.

### EXAMPLES

### Example 1. Cosmetic body composition according to the present invention.

**Table 1. Cosmetic body composition according to the present invention - preferred embodiment.**

| Ingredient | **% Total** |
|---|---|
| Water | 72.450 |
| Olus Oil (Cegesoft PS 6) | 4.998 |
| Tocopherol | 0.003 |
| Glycerin | 5.000 |
| Caprylic/Capric Triglyceride | 4.000 |
| Hydroxyethyl Acrylate/Sodium Acryloyldimethyl Taurate Copolymer | 1.200 |
| Squalane | 0.900 |
| Water | 0.615 |
| Polysorbate 60 | 0.240 |
| Sorbitan isostearate | 0.045 |
| *Ricinus communis* oil | 3.000 |
| Isoamyl Cocoate | 1.000 |
| CI 77891 | 0.979 |
| Alumina | 0.014 |
| Glycerin | 0.007 |
| Caprylic/Capric Triglyceride | 0.660 |
| Ammonium Acryloyldimethyltaurate/VP Copolymer | 0.274 |
| Trilaureth-4 phosphate | 0.038 |
| Polyglyceryl-2 sesquiisostearate | 0.028 |
| CI 77891 | 0.484 |
| CI 77491 | 0.221 |
| Cellulose acetate | 0.085 |
| Acrylates/ammonium methacrylate copolymer | 0.051 |
| Propylene Glycol Stearate | 0.042 |
| Magnesium Stearate | 0.017 |
| Phenoxyethanol | 0.800 |
| CI 77891 | 0.430 |
| CI 77492 | 0.196 |
| Cellulose acetate | 0.075 |

| Ingredient | **% Total** |
|---|---|
| Acrylates/ammonium methacrylate copolymer | 0.045 |
| Propylene Glycol Stearate | 0.038 |
| Magnesium Stearate | 0.015 |
| Tocopheryl Acetate | 0.500 |
| Perfume | 0.500 |
| Linalool | 0.034 |
| Benzyl alcohol | 0.000 |
| DMDM Hydantoin | 0.180 |
| Water | 0.120 |
| CI 77891 | 0.161 |
| CI 77499 | 0.074 |
| Cellulose acetate | 0.028 |
| Acrylates/ammonium methacrylate copolymer | 0.017 |
| Propylene Glycol Stearate | 0.014 |
| Magnesium Stearate | 0.006 |
| Xanthan Gum | 0.250 |
| Polyglyceryl-3 caprylate | 0.100 |
| Disodium EDTA | 0.100 |

### Example 2. Hydration

This benefit was evaluated through an objective, noninvasive methodology designated corneometry, which assesses the amount of water aggregated to the skin surface upon use of the product by evaluating the change in capacitance generated by such water build-up. In this evaluation measurements were taken prior to application of the product in order to provide information on the baseline condition of the skin, and after 15 minutes, 2 hours, 8 hours, 24 hours, 30 hours and 48 hours after application in order to identify the hydration improvement conferred by the product on the skin both immediately after use and throughout the day. Applications are made on the forearm, which is a region less affected by external factors and being more protected from sun exposure.

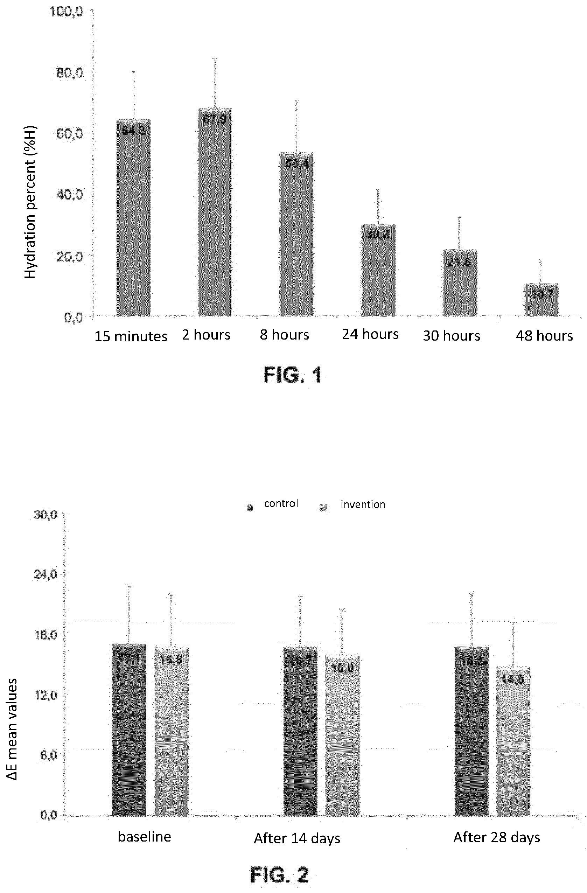

Figure 1 shows the skin hydration kinetics by corneometry, showing the percentage of hydration improvement provided by the cosmetic body composition according to the present invention as compared to the baseline skin condition (prior to application of the composition).

Figure 2 shows the mean values of the variation in transepidermal skin water loss (mean ± SD, n = 27), showing the hydration performance of the cosmetic body composition according to the present invention for up to 48 hours.

The cosmetic body composition according to the present invention has shown significantly positive results at all times evaluated hence increasing the skin hydration level by up to 67.9%. According to the study protocol and procedures employed to evaluate the increase in skin hydration, the following conclusions were made:
- the product under investigation conferred significantly increased skin hydration after 15 minutes, 2, 8, 24, 30 and 48 hours of application as compared to the control (skin without any products applied thereon).
- The product under investigation kept the skin hydrated for up to 48 hours after application.
- the product under investigation Natura Product Formula Code: 3349.27368.46 has increased the skin hydration level by up to 67.9%.
- 100% of the research participants have shown improvements in skin hydration after application of the product under investigation.

### Example 3. Barrier strengthening

This benefit was assessed using an objective, non-invasive methodology designated TEWL ("Transepidermal Water Loss") with 27 volunteers aged 32 to 56 years, which method indicates the skin barrier integrity through measurements of transdermal water loss that represents one of the primary barrier functions, that is, in addition to preventing the entry of microorganisms, chemicals and various forms of radiation, it prevents the body from losing water and nutrients while maintaining the skin impervious, which is essential for the proper functioning of the organism. In this evaluation measurements were taken prior to application of the product in order to provide information on the baseline condition of the skin, and after 14 and 28 days of home use of the product.

Applications are made on the forearm, which is a region less affected by external factors and being more protected from sun exposure.

The table below shows the percentage of barrier strengthening provided by the cosmetic body composition according to the present invention, when comparing the baseline condition of the skin (prior to application thereof) as well as the percentage of volunteers in which this effect was observed.

**Table 2. Skin barrier strengthening results**

| Days of Use | % improvement | % of volunteers who showed improvement |
|---|---|---|
| 14 | 3.1% | 81% |
| 28 | 10.1% | 100% |

According to the obtained results, the product under investigation applied on the volar forearm skin has conferred a significant skin barrier strengthening effect after 14 and 28 days of home use as compared to the control. The percent skin barrier strengthening values relative to the baseline skin condition and the control were 3.1% and 10.1% after 14 and 28 days of home use, respectively. 100% of the research participants have shown strengthened skin barrier after using the product under investigation at home.

According to the study protocol and procedures used to assess the skin barrier strengthening effect provided by applying the product under investigation on the forearm skin, it was found that:
- it has conferred significant skin barrier strengthening effect after 14 and 28 days of home use as compared to control (skin without any products being applied thereon).
- the percent skin barrier strengthening values relative to the baseline skin condition and the control were: 3.1% after 14 days and 10.1% after 28 days of home use.
- 100% of the research participants have shown strengthened skin barrier after using the product under investigation at home.

### Example 4. Skin Color Evenness/Homogenization/ Masking of Imperfections

This benefit was evaluated by an objective, noninvasive methodology consisting of performing color evaluations of high resolution digital photographs obtained with a digital camera in a software. Evaluations are based on high resolution digital photographs obtained under controlled conditions at the beginning of the study and after 15 minutes of the application of the product under investigation. Skin color evenness was calculated as the percentage reduction in skin color variation after application relative to baseline.

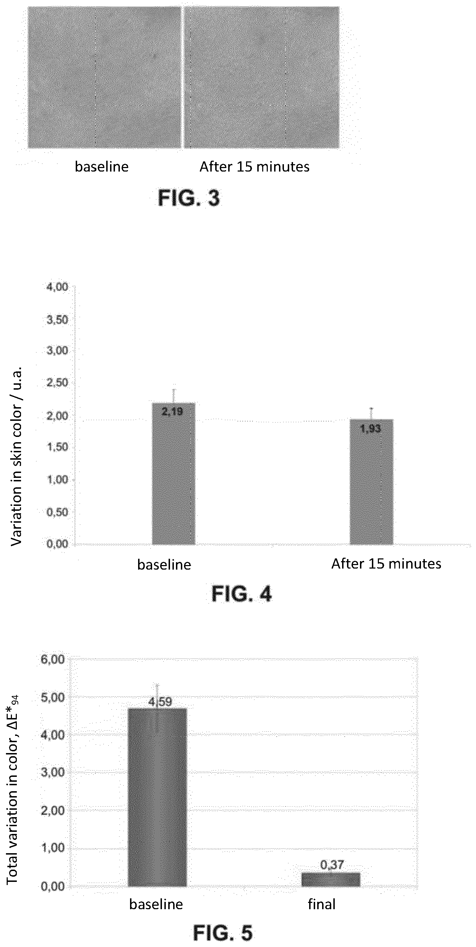

Figure 3 shows the skin color evenness provided by the use of the cosmetic composition according to the present invention. Skin color evenness was calculated as the percentage reduction in skin color variation after application relative to baseline.

Figure 4 shows the variation in baseline skin color and after 15 minutes of applying the cosmetic body composition according to the present invention.

The cosmetic body composition according to the present invention has shown a significant increase of 11.6% sometimes reaching 13.7% in skin color evenness through optical masking after 15 minutes of application. In addition, 100% of the research participants have shown improvements in skin color evenness after 15 minutes of application of the product under investigation.

According to the study protocol and procedures employed to assess the optical masking provided by the product under investigation, the following conclusions were made:
- the product under investigation has shown a significant increase of 11.6% sometimes reaching 13.7% in skin color evenness through optical masking after 15 minutes of application.
- 100% of the research participants have shown improvements in skin color evenness after application of the product under investigation.

### Example 4. Fabric stain

This benefit has been assessed using an *in vitro* methodology consisting of using a "Byk-Gardner Spectro-Guide Sphere Gloss" spectrophotometer to assess the fabric color. In this methodology 10 samples of white, 100% cotton fabric are read with the spectrophotometer "Byk-Gardner Spectro-Guide Sphere Gloss" before application of the product so as to know the color condition of the fabric at baseline, after application and drying the product in a standardized environment at 55 ± 5% relative humidity and 22 ± 2 °C for 2 hours (initial reading) and after washing and drying (in a standardized environment at 55 ± 5% relative humidity and 22 ± 2 °C for 24 hours) under standardized conditions (final reading).

For determining stains on the fabric, it is considered whether or not the stain remains after washing using the CIE94 color system, where the color difference, designated ΔE*94, is considered as visually noticeable when it reaches values above 1.0.

Figure 5 shows results of total color variation ΔE*94 (calculated relative to the standard sample) of fabric samples after application of the cosmetic body composition according to the present invention and after washing.

It can be concluded that the product under investigation does not stain fabrics, as the washing procedure has removed the stain present on the fabric.

### Example 5. Assessment of perceived efficacy

This benefit was assessed using a subjective methodology whereby 34 volunteers were asked to respond "yes" to "no" to questions made after 21 days of use of the product:
- Did you notice that the product brightened your skin?
- Did you notice that the product left your skin with more vitality (life)?
- Did you notice that the product has improved the overall appearance of your skin?

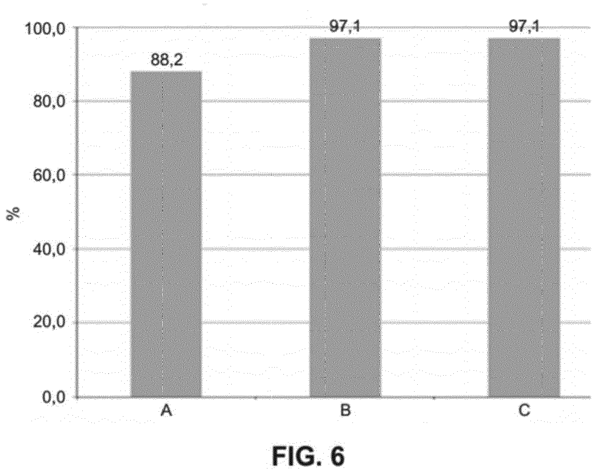

Figure 6 shows the percentage of acceptance by volunteers in terms of the attributes assessed after using the cosmetic body composition according to the present invention.

After 21 days using the product according to the present invention the following perceived efficacy results were obtained:
- 88.2% of the participants answered "yes" to the question "Did you notice that the product brightened your skin?
- 97.1% of participants answered "yes" to the question "Did you notice that the product left your skin with more vitality (life)?"
- 97.1% of the participants answered "yes" to the question "Did you notice that the product has improved the overall appearance of your skin?"

### Example 6. Product safety results

To assess the absence of sensations and signs of discomfort, safety evaluation tests were performed under the supervision of dermatologists. Safety of the product according to the present invention has been demonstrated by means of a study of acceptability in use and HRIPT.

Acceptability in use: this study aims to verify the acceptability of a topical cosmetic product by confirming the absence of adverse events and sensations of skin and eye discomfort (in the case of products intended for use on the face) when applied as recommended and accompanied by a dermatologist and ophthalmologist.

It consists of volunteers using the test product at home under real use conditions (the same condition as shown on the product packaging) in order to verify whether the product is well accepted by the volunteers' skin. Dermatological and opthalmic clinical evaluations are performed prior to the use of the test product (to verify the volunteer's initial skin and eye condition and whether he/she is able to participate in the study) and after 21 days of use to ensure that the product did not promote any changes on the skin and eye health.

As a result, the product was shown to be safe for use in humans on the area recommended by the manufacturer, causing no rash or sensitization reactions in the sample of volunteers analyzed herein, being declared as Dermatologically tested.

HRIPT: This study aims to demonstrate the absence of primary skin irritation, cumulative skin irritation and the potential of skin sensitization of the product under maximized conditions, taking into account the amount of product applied and the occlusion procedure (use of occlusive and semi-occlusive dressings) so as to capture possible feelings of discomfort and/or clinical signs that the product may have a cumulative effect on the skin, characterizing possible irritation and/or sensitization reactions.

As a result, the product can be deemed safe under the assessed conditions as it did not induce primary or cumulative skin irritation or skin sensitization in the study group.

From the teachings provided in the text and the examples presented herein the person skilled in the art will readily appreciate the advantages of the invention and will be able to propose variations and equivalent embodiment alternatives without departing from the scope of the invention as defined in the appended claims.

## Claims

1. A COSMETIC BODY COMPOSITION **characterized in that** it comprises:
(a) at least one ingredient that provides essential nutrients to the skin; and
(b) at least one encapsulated pigment.

2. The COMPOSITION of Claim 1, **characterized in that** the ingredient that provides essential nutrients to the skin is selected from emollients, humectants, conditioners and/or antioxidants.

3. The COMPOSITION of Claim 2, **characterized in that** the ingredient that provides essential nutrients to the skin is selected from C₈-C₁₀ triglyceride, C₁₆-C₁₈ triglyceride, tocopheryl acetate, squalene and/or glycerin.

4. The COMPOSITION of Claim 1, **characterized in that** the ingredient that provides essential nutrients to the skin is present in an amount of from 0.1 to 20% by weight relative to the total weight of the composition.

5. The COMPOSITION of Claim 1, **characterized in that** the encapsulated pigment comprises cellulose acetate, ammonium methacrylate copolymer, magnesium stearate and/or propylene glycol stearate.

6. The COMPOSITION of Claim 1, **characterized in that** the encapsulated pigment comprises CI 77491, CI 77492, CI 77499 and/or CI 77891.

7. The COMPOSITION of Claim 6, **characterized in that** the ingredient that provides essential nutrients to the skin is present in an amount of from 0.1 to 3% by weight relative to the total weight of the composition.

8. A BODY MOISTURIZER, **characterized in that** it comprises the cosmetic body composition of any one of claims 1 to 7, and cosmetically acceptable excipients.

9. The USE OF THE COSMETIC BODY COMPOSITION of any one of claims 1 to 7, **characterized in that** it is in the manufacture of a body moisturizer useful to moisturize, strengthen the skin barrier and/or even the skin tone.

10. A COSMETIC METHOD OF EVENING THE SKIN TONE, **characterized in that** it consists of applying to the body region in need a cosmetically effective amount of the cosmetic composition as defined in any one of claims 1 to 7 until it is absorbed by the skin.
